# EUROPEAN PATENT APPLICATION

(11) **EP 3 627 154 A1**
(43) Date of publication of application: **25.03.2020**
(21) Application number: 18195605.3
(22) Date of filing: 20.09.2018
(51) Int. Cl.: G01N 33/569

(54) **PROCESS FOR INCREASING THE SENSITIVITY OF AN IMMUNOLOGICAL TEST FOR DETECTING AN ANTIGEN IN A SAMPLE**

(71) Applicant: Mikrogen GmbH, 82061 Neuried (DE)
(72) Inventor: Pfister, Heiko, 86504 Merching (DE); Böcher, Oliver, 86862 Lamerdingen (DE)
(74) Representative: Keller, Günter

(57) **Abstract**

The present invention relates to a process for increasing the sensitivity of an immunological test for detecting an antigen in a sample
wherein the solid support carrying the antibody specific for the antigen to be detected is contacted with the sample containing the antigen and after a sufficient time of reaction this sample is removed
wherein the same solid support carrying the antibody specific for the antigen is in the course of a repetitive application of the sample again contacted with a fresh aliquot of the sample and after a sufficient reaction time removed
whereby the step of repetitive application of the sample containing the antigen is repeated 1 to 5 times finally followed by washing the solid support with a suitable washing solution.

## Description

Infection with a microbial or viral pathogen can be diagnosed in many cases by the detection of pathogen-specific markers in body fluids as blood, urine, etc. Such markers may be antibodies which are formed by the host organism in response to the infection of the microorganism. Alternatively nucleic acids may be detected. Another way of diagnosing an infection is to detect an antigen derived from the microorganism. Such antigen may be a characteristic molecule of the microorganism, for example a surface protein, a lipopolysaccharide or other polysaccharide-containing molecules like galactofuranose antigens which may be released from the microorganism into the host organism. The detection of specific marker molecules also plays an important role in oncological diagnostics.

The detection of traces of an infective microorganism is frequently performed by the detection of nucleic acids in the sample, in particular by PCR technology. There are, however, some cases where the PCR technology is not reliable, for example in stages where only a few copies of the nucleic acid of the infective agent are present in the sample. In such cases it may be advisable to perform the diagnosis on an immunological basis. There may be some stages of the infection where not enough antibodies have been formed by the infected host and in those cases, it may be the best choice to detect the antigen in the sample.

Since the concentration of the antigen in the sample may be, however, very low, highly sophisticated methods of detection may be required. One of the preferred embodiments for detecting an antigen in a sample is a sandwich ELISA test. In such sandwich ELISA tests polyclonal antibodies or monoclonal antibodies are attached to a solid support, for example on the wells of a microtiter plate. Alternatively the antibodies may also be attached to beads or stripes.

Normally the sample is contacted with the antibodies attached to the solid support and the antigen binds to the antibodies. After a washing step the antigen which is bound to the antibody attached to the solid support is detected by reaction with another antibody which is also specific for the antigen. The selection of suitable antibodies which bind to the antigen depends on the specific circumstances. Preferably the antibodies do not bind to the same epitope of the antigen. When monoclonal antibodies are used it is preferred to use one antibody which attaches the antigen to the solid support and which binds to a specific epitope. The detection antibody should bind to another epitope which is preferably not located in close neighborhood of the epitope to which the other antibody binds.

The selection of the appropriate antibody depends on the structure of the antigen to be detected. When the antigen for example comprises repetitive structures, which form epitopes, it may be preferred to use the same (monoclonal) antibody against such an epitope for the purpose of the capture antibody and the detection antibody as well. In other embodiments, when there are for example no repetitive epitopes structures, it may be preferable that the (monoclonal) capture antibody binds to an epitope, which is, however, not the same epitope to which the detection antibody binds.

When polyclonal antibodies are used the binding of the antibodies to the antigen occurs in a random distribution. It is also preferred to use a combination of one or more monoclonal antibodies with polyclonal antisera. The detection antibody is usually coupled with a moiety which causes a signal. This can be for example horse radish peroxidase which is able to form a colored product from a precursor. The degree of conversion from the precursor to the colored product corresponds to the amount of the antigen in the sample.

Some critical infections occurring after organ transplantation are frequently caused by microorganisms which are for healthy patients without severe risk. Examples are fungi, or viruses like herpes simplex or Epstein Barr Virus. Patients who received an organ transplantation, in particular kidney, liver, heart or lung from a donor are at risk of an infection which may result in the death of the patient since usually an organ transplantation involves an immune suppression.

It is therefore of extreme importance to detect a potential infection as soon as possible in order to initiate suitable countermeasures which may save the life of the patient. One of the dangerous infections is caused by *Aspergillus.* Normally *Aspergillus* is not dangerous for healthy patients, but for freshly transplanted patients, however, *Aspergillus* may be a deadly threat.

In the case of an infection with the mold *Aspergillus,* a component of the cell wall (Galactomannan, GM) may be used as a pathogen marker. With a marker-specific antigen-sandwich ELISA facilitating GM-specific antibodies invasive aspergillosis can be diagnosed in an early stage and monitored during therapy.

Another example is an infection with a high-risk type of the human papilloma virus (HPV) which may cause cervical neoplasia with the potential to progress to cervical cancer. The viral protein E7 of HPV may be used as a prognostic marker for disease progression.
For both conditions, infectious diseases and/or oncologic disorders, a high sensitivity of the assay is required in order to detect the relevant marker which is present only in very low concentrations.

It is therefore an object of the present invention to provide a method with increased sensitivity for detecting an antigen which is present in only small quantities in the sample.

The present invention provides a process for increasing the sensitivity of an immunological test for detecting an antigen in a sample. In this process a solid support carrying antibodies which are specific to the antigen to be detected is used. The antibodies are attached to a solid support which may be the wells of a microtiter plate or the bottom thereof or beads to which the antibodies are attached. Those antibodies attached to the solid support are then contacted with the sample containing the antigen and the mixture is allowed to react for a sufficient period of time. Frequently the sample is agitated and stirred at room temperature. After a sufficient period of time the sample is removed and the solid support is washed with a suitable washing solution. The washing buffer is composed in such a manner that unspecific impurities are washed away while the antigen bound to the specific antibody remains attached.

In the process according to the invention the solid support carrying the antibodies specific for the antigen is again contacted with a fresh aliquot of the sample containing the antigen. The sample and the antibodies are allowed to react for an appropriate period of time which may range from 10 minutes to 120 minutes, preferably 20 minutes to 40 minutes, in particular 30 minutes, and then the solid support carrying the antibody to which the antigen is bound is washed again. This step of repetitive application of the sample can be repeated several times. In a preferred embodiment the antibody and the sample containing the antigen are contacted and optionally washed thereafter 2-5 times, whereby in a particularly preferred embodiment the repetitive application is performed such that fresh aliquots of the sample are brought into contact with the antibodies 4 times.

It is an important aspect of the present invention that the process produces best results when the antigen is present in the sample only in low concentration. Such low concentrations of the antigen to be detected occur e.g. in the case of human papilloma viruses which may cause cervical cancer or aspergillosis.

According to the invention it is possible to detect an antigen which is present in a very low concentration which may range from 0.01 ng to 100 ng per ml of test sample, preferably from 0.01 ng to 10 ng, and more preferred from 0.1 ng to 2.0 ng antigen per ml of test sample.

In order to obtain the best sensitivity the parameters of the test should be optimized. It has, however, to be taken into account which molecular configuration the antigen has. Depending on the molecular structure of the antigen like e.g. small size of the antigen or not repetitive structure of the antigen it may be difficult to reach the desired sensitivity. In such cases it may be helpful to optimize the test condition for example by adding a suitable detergent or increasing the salt concentration at the appropriate steps of the detection process.

It has been found that in particular when the antigen is present in the sample in a very low concentration that the repetitive application of the sample to the test kit allows a sensitivity which is higher than applying a larger amount of the sample to the antibodies in one batch. The higher the concentration of the antigen in the sample is, the lower the synergistic effect resulting in increased sensitivity is.

The process according to the present invention is also preferably used to detect an infection caused by the mold *Aspergillus* whereby a particularly preferred test principle can be described as follows:
*Aspergillus* galactomannan specific antibody AB-131 is coated onto a microtiter plate at 1 µg/well serving as capture antibody. When a patient sample is applied (e.g. 2 h at room temperature) to the plate the galactomannan is bound by the capture antibody. After washing away unbound material captured antigen is detected colorimetrically or luminometrically by the same galactomannan specific antibody conjugated to horseradish peroxidase.

Since it is difficult to evaluate an immunological test with sera obtained from patients the tests described herein have been prepared with sera obtained from healthy volunteers whereby the sera were spiked with well-defined small amounts of the antigen (*Aspergillus* galactomannan). In order to show the superior sensitivity of the process, serum samples were generated by spiking *Aspergillus* galactomannan (approx. 0,75 ng/ml low positive - approx. 1,5ng/well medium positive - approx. 3ng/ml high positive) into serum from a healthy blood donor at different dilutions. Serum without galactomannan served as negative control.

Galactomannan was collected from the supernatant of an *Aspergillus fumigatus* wildtype liquid culture. *Aspergillus fumigatus* wildtype was obtained from the culture collection and grown in medium for about two weeks. The supernatant of the liquid culture was filtrated and the galactomannan antigen was concentrated and purified to a final concentration of approx. 30µg/ml. The galactomannan-containing preparation was used for spiking the serum samples.

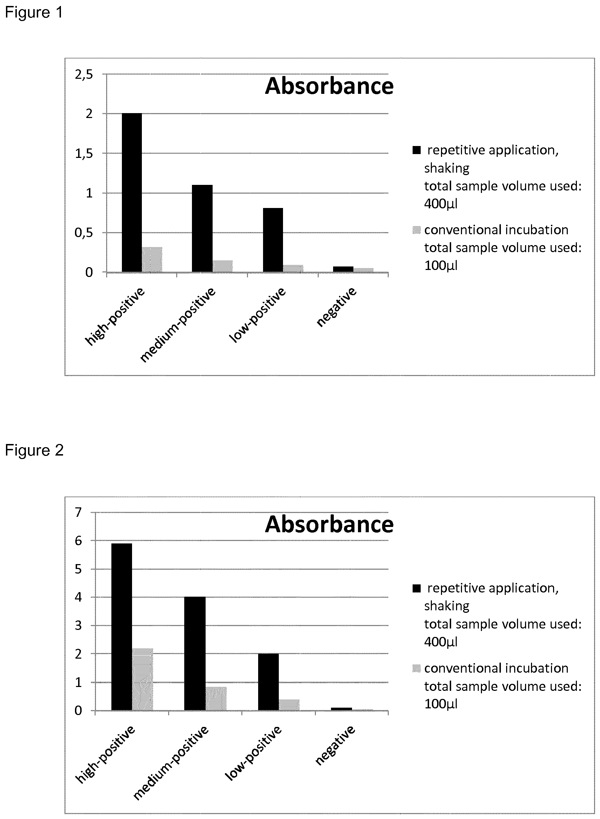

Serum samples were heat treated in the presence of EDTA before application to the assay to precipitate potentially interfering serum proteins. For the EDTA treatment the serum samples were mixed with EDTA-containing buffer and then stored at a temperature of approx. 120°C for 5-10 minutes. The formed precipitate was separated from the sample by centrifugation and the clear supernatant was used in the test according to the present invention. Figure 1 shows clearly that the surprising synergistic effect can be observed best with low positive samples. In example 1 an amplification of the signal of 8.6 could be obtained with low positive samples whereas with high positive samples also an amplification could be observed which was, however, only 6.2-fold.

For the process according to the invention material of the same specimen was repeatedly applied to the ELISA plate (preferably 4 x 30 min at room temperature). During specimen incubation the plate was continuously shaken on a conventional table top microplate shaker at approx. 500-600 rpm at a 2,2 mm amplitude.

This step eliminates the need for enrichment of the analyte by ultrafiltration, precipitation or other methods. This process step is easily reproducible and scalable. When repetitive application is combined with shaking of the assay plate during sample incubation, a multiple signal amplification has been achieved.

In the linear range of ELISA assays the signal is proportional to the analyte concentration whereas very low and very high analyte concentrations yield sub proportional signals typically resulting in a sigmoidal curve if analyte concentration is plotted against the assay signal.

Assuming ideal conditions, a 4-fold application of an analyte to the assay plate may maximally yield a 4-fold signal increase. Alternatively, if the double amount of a patient sample is applied, a maximal signal increase by a factor of 2 would be expected.
The present invention is illustrated by the following experiments:

### Example 1

Comparison of conventional method (incubation of human sample 2 h) vs. invention (incubation of human sample 4 x 30min plus shaking)

A high, medium and low GM-positive sample was generated by spiking human serum with Aspergillus Galactomannan. The same serum without GM served as negative control.

Incubating samples 4 x 30' with shaking instead of 2h incubation on the lab bench amplifies the signal 6.2 (high positive sample)-8.6 (low positive sample) fold.

The results are shown in Figure 1.

### Example 2

The experiment was performed identical to experiment 1 but using a commercially purchased anti-GM coated ELISA-plate taken from a Platelia™ Aspergillus Ag-ELISA Kit (Bio-Rad).

A high, medium and low GM-positive sample was generated by spiking human serum with Aspergillus Galactomannan. The same serum without GM served as negative control.

Incubating samples 4x30' with shaking instead of 2h incubation on the lab bench amplifies the signal 2.7 (high positive sample)-4.9 (low positive sample) fold.

The GM titration curve demonstrates a proportional linear relationship between GM concentration and ELISA signal for absorbance values below OD 4, probably because of non-overlapping epitopes between antibody conjugate and capture antibody. A 4.9-fold increase of the low positive sample thus corresponds to a 20% higher signal than expected by an additive effect. However, the effect of shaking has not been tested separately.

The results are shown in Figure 2. Example 2 shows that the surprising effect obtainable by repetitive application of the sample can be observed also with commercially available test kits. Also with this test kit component a surprising increase of the sensitivity could be observed with low positive samples whereas also with high positive samples an increase of the sensitivity could be observed which was, however, not as strong as the effect obtained with low concentration samples.

### Example 3

Comparative titration of galactomannan spiked into serum from a blood donor: 100µl sample (incubation 2 h plus shaking) vs. invention (incubation of human sample 4x30min plus shaking). The same serum without GM served as negative control.

When incubating samples 4x30' with shaking the measured relative light units (RLU; using a chemiluminescent substrate) correlate approximately linearly with the analyte concentration over the entire measuring range from 1:5.000 to 1:640.000 dilution of galactomannan (dashed blue line in double logarithmic plot). Plotting the data from the comparative experiment with 2h shaking yields a baseline asymptotic curve with an approximated transition point from asymptote to a linear signal to analyte correlation between 1:20.000 - 1:40.000 dilution of galactomannan. Compared to single application, the new method amplified the signal-3.23 to 23,09 times over the entire measured range. A maximum of 4 times may be expected under optimal conditions from the difference in cumulated analyte amount.

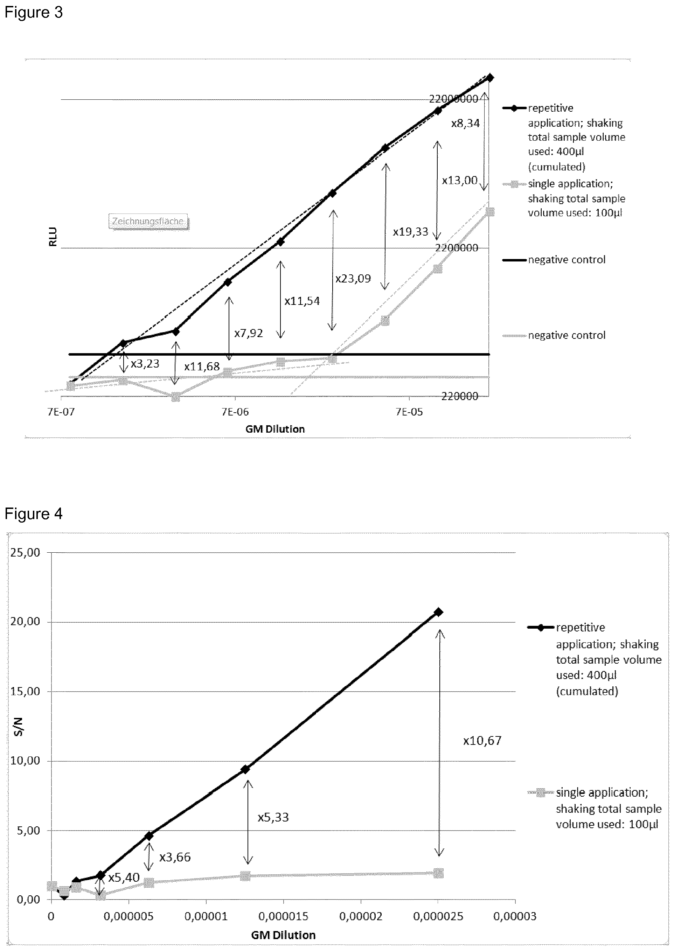

The results are shown in Figure 3. This is clear evidence for the surprising superior sensitivity.

Compared to a single application of the sample, the method according to the invention improved the signal-to-noise ratio (S/N; calculated from the measured signal divided by negative control value) 3.66 to 10.67 times with low dose GM dilutions. Less than 4 times may be expected under optimal conditions from the difference in cumulated analyte amount. This is shown in Figure 4.

### Example 4

Comparison of alcohol precipitated sample (incubation of human sample 2 h) vs. invention (incubation of human sample 4x30min plus shaking)

A high, medium and low GM-positive sample was generated by spiking human serum with Aspergillus Galactomannan. The same serum without GM served as negative control. Alcohol precipitation was performed to concentrate GM in the test sample. The applied amount of resolved precipitate corresponds to approx. 670 µl sample.

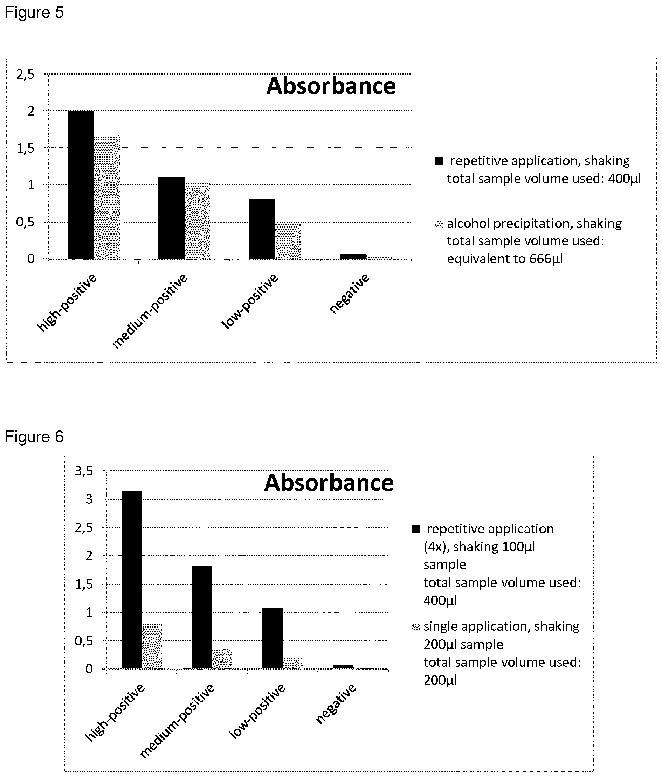

Incubating samples 4 x 30' with shaking instead of 2 h shaking resolved alcohol precipitate yields a 10% (medium positive sample)-70% (low positive sample) stronger signal although approx.40% less sample was used. The results are shown in Figure 5.

### Example 5

Comparison of incubation with 200 µl sample volume 2 h plus shaking vs. incubation of 100 µl sample 4x30 min plus shaking.

A high, medium and low GM-positive sample was generated by spiking human serum with Aspergillus Galactomannan. The same serum without GM served as negative control.

Incubating samples 4 x 30' with shaking (100 µl samples) instead of incubating 200 µl sample 2 h with shaking amplified the signal 3.9 (high positive sample)-5.0 (medium positive sample) fold. A maximal signal difference of a factor of 2 between both methods could be calculated from the applied analyte amounts. The results are shown in Figure 6.

**Conclusion:** The new method of the present invention increases the technical/analytical sensitivity of an antigen sandwich ELISA, especially in the low-dose range, which is of particular relevance for diagnostics. It clearly outperforms simple multiplying of the sample size. The invention thus generates amplified ELISA signals that cannot be explained by an additive effect proportional to the analyte concentration. The principle of the present invention has been shown with galactomannan derived from *Aspergillus fumigatus* as model antigen. The same effect can be observed, however, with other antigens. Experiments have also been performed with other antigens and the same surprising increase in sensitivity, in particular at low concentrations of the antigen has been observed.

## Claims

1. A process for increasing the sensitivity of an immunological test for detecting an antigen in a sample
wherein the solid support carrying the antibody specific for the antigen to be detected is contacted with the sample containing the antigen and after a sufficient time of reaction this sample is removed
**characterized in that** the same solid support carrying the antibody specific for the antigen is in the course of a repetitive application of the sample again contacted with a fresh aliquot of the sample and after a sufficient reaction time removed
whereby the step of repetitive application of the sample containing the antigen is repeated 1 to 5 times finally followed by washing the solid support with a suitable washing solution.

2. Process according to claim 1 wherein the step of repetitive application of the sample is repeated 2 to 3 times.

3. Process according to claim 1 or 2 **characterized in that** the antigen to be detected in the sample is present in a low concentration only.

4. Process according to any of claims 1 to 3 **characterized in that** the antigen is present in a concentration of 0,01 ng to 10 ng per ml of sample.

5. Process according to claim 4 **characterized in that** the antigen is present in a concentration of 10ng to 1µg per ml of sample.

6. Process according to any of claims 1 to 5 **characterized in that** the test is a sandwich ELISA test.

7. Process according to any of claims 1 to 6 **characterized in that** the repetitive application of the sample is performed under shaking of the reaction mixture.

8. Process according to claim 7 **characterized in that** the gentle shaking of the reaction mixture is performed for at least 30 minutes in each repetitive application of the sample.

9. Process according to any of claims 1 to 8 **characterized in that** the antigen is derived from a microbial, fungal or viral pathogen.

10. Process according to claim 9 **characterized in that** the antigen is derived from the species of Aspergillus.

11. Process according to claim 10 **characterized in that** the antigen is a galactomannan derived from a species of *aspergillus.*

12. Process according to any of claims 1 to 11 **characterized in that** it is an ELISA test.
